# EUROPEAN PATENT APPLICATION

(11) **EP 4 600 646 A1**
(43) Date of publication of application: **13.08.2025**
(21) Application number: 23874969.1
(22) Date of filing: 06.10.2023
(51) Int. Cl.: G01N 33/53, A61K 45/00, A61P 17/00, A61P 17/04, A61P 29/00, A61P 37/08, C07K 16/28, C12Q 1/68, G01N 33/50, G01N 33/68

(54) **METHOD FOR DETECTING INFLAMMATORY SKIN DISEASE AND JUDGING SEVERITY THEREOF**

(30) Priority: 07.10.2022 JP 2022162538; 02.03.2023 JP 2023031988
(71) Applicant: Nanoegg Research Laboratories, Inc., Tokyo 170-0013 (JP)
(72) Inventor: KITAZAWA, Kazuyuki, Kawasaki-shi, Kanagawa 210-0821 (JP); TANAKA, Kazunori, Kawasaki-shi, Kanagawa 210-0821 (JP)
(74) Representative: Moré, Solveig Helga
(86) International application number: PCT/JP2023/036615
(87) International publication number: WO 2024/075844

(57) **Abstract**

The present disclosure provides a technique for examining the acquisition risk of an inflammatory skin disease, the severity or the degree of progression thereof. The present disclosure provides a method for analyzing a biological sample collected from a test subject to examine the presence/absence of the acquisition of an inflammatory skin disease, the acquisition risk, the severity and/or the degree of progression thereof in the test subject, the method including a step for measuring the amount and/or concentration of an epithelial-mesenchymal transition marker in the biological sample collected from the test subject.

## Description

### TECHNICAL FIELD

The present invention relates to a method for testing the presence or absence, likelihood of the presence, the severity and/or the degree of progression of an inflammatory skin disease, and a reagent and diagnostic composition therefor.

### BACKGROUND ART

There are various types of skin diseases involving inflammation, and for example, in atopic dermatitis, it is known that symptoms progress by inflammation.

As a method for testing atopic dermatitis, a method for measuring the amount of TARC (thymus and activation-regulated chemokine) and the amount of SCCA2 (squamous cell carcinoma antigen 2) in blood is used, but in clinical practice, a more sensitive biomarker for testing the presence or absence, likelihood of the presence, severity and/or degree of progression of atopic dermatitis is desired. In addition, there are many pediatric patients with atopic dermatitis, and thus it may be difficult to collect blood as a biological sample.

### SUMMARY OF THE INVENTION

### PROBLEMS TO BE SOLVED BY THE INVENTION

An object of the present disclosure is to provide a minimally invasive and simple test technique for measuring the presence or absence, likelihood of the presence, the severity and/or the degree of progression of an inflammatory skin disease such as atopic dermatitis.

### SOLUTIONS TO THE PROBLEMS

As a result of intensive investigations in view of the above problem, the present inventor has found that it is possible to test, diagnose, or predict the presence or absence, likelihood of the presence, the severity and/or the degree of progression of an inflammatory skin disease by a method including a step of measuring the amount and/or concentration of an epithelial-mesenchymal transition marker in a biological sample collected from a subject. As a result of further research based on this finding, the present inventor has completed the invention of the present disclosure. That is, the present invention encompasses the following aspects.

### (Item 1)

A method for analyzing a biological sample collected from a subject for testing presence or absence, likelihood of presence, severity, and/or degree of progression of an inflammatory skin disease in the subject, the method including a step of measuring an amount and/or concentration of an epithelial-mesenchymal transition marker in the biological sample collected from the subject.

### (Item 2)

The method according to the above item, wherein the inflammatory skin disease is atopic dermatitis.

### (Item 3)

The method according to any one of the above items, wherein the epithelial-mesenchymal transition marker is at least one selected from the group consisting of N-cadherin, vimentin, Twist, and Snail.

### (Item 4)

The method according to any one of the above items, further including a step of comparing a preset reference value with an amount and/or concentration of an epithelial-mesenchymal transition marker in a biological sample collected from the subject.

### (Item 5)

The method according to any one of the above items, wherein the reference value is an amount and/or concentration of an epithelial-mesenchymal transition marker in a biological sample collected from a subject not having an inflammatory skin disease.

### (Item 6)

The method according to any one of the above items, wherein the biological sample is a sample of skin or derived from skin.

### (Item 7)

The method according to any one of the above items, wherein the sample derived from skin is at least one selected from the group consisting of a horny layer, an epidermis, and a hair follicle.

### (Item 8)

A testing agent including a detection reagent for an epithelial-mesenchymal transition marker, for testing presence or absence, likelihood of presence, severity, and/or degree of progression of an inflammatory skin disease in a subject.

### (Item 9)

The testing agent according to any one of the above items, wherein the detection reagent is selected from the group consisting of an antibody or an antigen-binding fragment thereof against the epithelial-mesenchymal transition marker, and a functional nucleic acid.

### (Item 10)

The testing agent according to any one of the above items, wherein the epithelial-mesenchymal transition marker is at least one selected from the group consisting of **N-**cadherin, vimentin, Twist, and Snail.

### (Item 11)

The testing agent according to any one of the above items, wherein the inflammatory skin disease is atopic dermatitis.

### (Item 12)

A composition for determining presence or absence, likelihood of presence, severity, and/or degree of progression of an inflammatory skin disease in a subject, the composition including a reagent for measuring an amount and/or concentration of an epithelial-mesenchymal transition marker.

### (Item 13)

A composition for treating or preventing an inflammatory skin disease, the composition including a molecule that inhibits epithelial-mesenchymal transition.

### (Item 1A)

A method for testing presence or absence, likelihood of presence, severity, and/or degree of progression of an inflammatory skin disease in a subject, the method including: a step of collecting a biological sample from the subject; a step of measuring an amount and/or concentration of an epithelial-mesenchymal transition marker in the biological sample; and a step of determining presence or absence, likelihood of presence, severity, and/or degree of progression of an inflammatory skin disease in the subject based on an amount and/or concentration of the epithelial-mesenchymal transition marker.

### (Item 2A)

The method according to the above item, wherein the inflammatory skin disease is atopic dermatitis.

### (Item 3A)

The method according to any one of the above items, wherein the epithelial-mesenchymal transition marker is at least one selected from the group consisting of N-cadherin, vimentin, Twist, and Snail.

### (Item 4A)

The method according to any one of the above items, further including a step of comparing a preset reference value with an amount and/or concentration of an epithelial-mesenchymal transition marker in a biological sample collected from the subject.

### (Item 5A)

The method according to any one of the above items, wherein the reference value is an amount and/or concentration of an epithelial-mesenchymal transition marker in a biological sample collected from a subject not having an inflammatory skin disease.

### (Item 6A)

The method according to any one of the above items, wherein the biological sample is a sample of skin or derived from skin.

### (Item 7A)

The method according to any one of the above items, wherein the sample derived from skin is at least one selected from the group consisting of a horny layer, an epidermis, and a hair follicle.

### (Item 8A)

A method for treating or preventing an inflammatory skin disease in a subject, the method including administering to the subject a therapeutically effective amount of a molecule that inhibits epithelial-mesenchymal transition.

### (Item 1B)

A detection reagent for an epithelial-mesenchymal transition marker for testing the presence or absence, likelihood of presence, severity, and/or degree of progression of an inflammatory skin disease in a subject.

### (Item 2B)

The detection reagent according to any one of the above items, wherein the detection reagent is selected from the group consisting of an antibody or an antigen-binding fragment thereof against the epithelial-mesenchymal transition marker, and a functional nucleic acid.

### (Item 3B)

The detection reagent according to any one of the above items, wherein the epithelial-mesenchymal transition marker is at least one selected from the group consisting of N-cadherin, vimentin, Twist, and Snail.

### (Item 4B)

The detection reagent according to any one of the above items, wherein the inflammatory skin disease is atopic dermatitis.

### (Item 5B)

A reagent for measuring an amount and/or concentration of an epithelial-mesenchymal transition marker for determining the presence or absence, likelihood of presence, severity, and/or degree of progression of an inflammatory skin disease in a subject.

### (Item 6B)

A molecule inhibiting epithelial-mesenchymal transition for treating or preventing inflammatory skin diseases.

### (Item 7B)

The use of a detection reagent for an epithelial-mesenchymal transition marker in a method for testing the presence or absence, likelihood of presence, severity, and/or degree of progression of an inflammatory skin disease in a subject.

### (Item 8B)

The use according to any one of the above items, wherein the detection reagent is selected from the group consisting of an antibody or an antigen-binding fragment thereof against the epithelial-mesenchymal transition marker, and a functional nucleic acid.

### (Item 9B)

The use according to any one of the above items, wherein the epithelial-mesenchymal transition marker is at least one selected from the group consisting of N-cadherin, vimentin, Twist, and Snail.

### (Item 10B)

The use according to any one of the above items, wherein the inflammatory skin disease is atopic dermatitis.

### (Item 11B)

The use of a reagent for measuring an amount and/or concentration of an epithelial-mesenchymal transition marker in a method for determining the presence or absence, likelihood of presence, severity, and/or degree of progression of an inflammatory skin disease in a subject.

### (Item 12B)

The use of a molecule inhibiting epithelial-mesenchymal transition in production of a medicament for treating or preventing an inflammatory skin disease.

### ADVANTAGES OF THE INVENTION

The present disclosure can test the presence or absence, likelihood of the presence, the severity, and/or degree of progression of the inflammatory skin disease in a minimally invasive and simple manner.

### BRIEF DESCRIPTION OF THE DRAWINGS

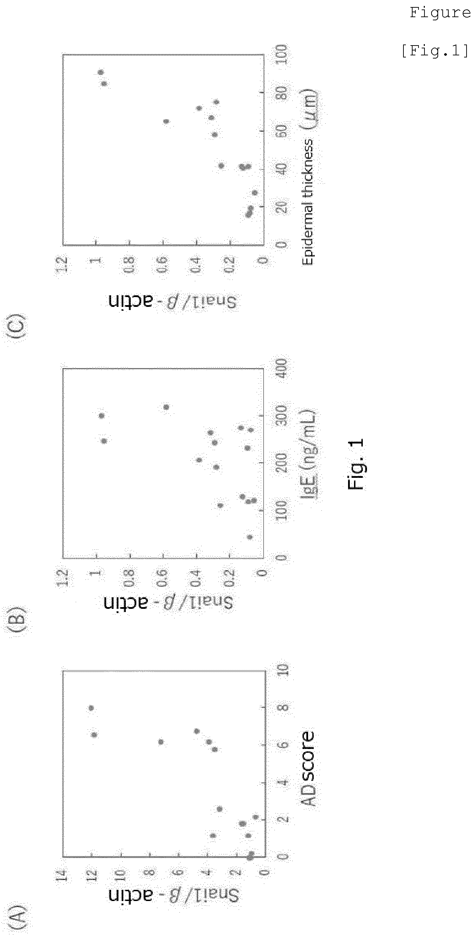

Fig. 1 shows the correlation between Snai1/β-actin and the severity of atopic dermatitis ((A) AD score, (B) IgE, and (c) epidermal thickness), as measured in Example 1. The vertical axis indicates the Snai1/β-actin value. The horizontal axis indicates the index value of the severity of atopic dermatitis.

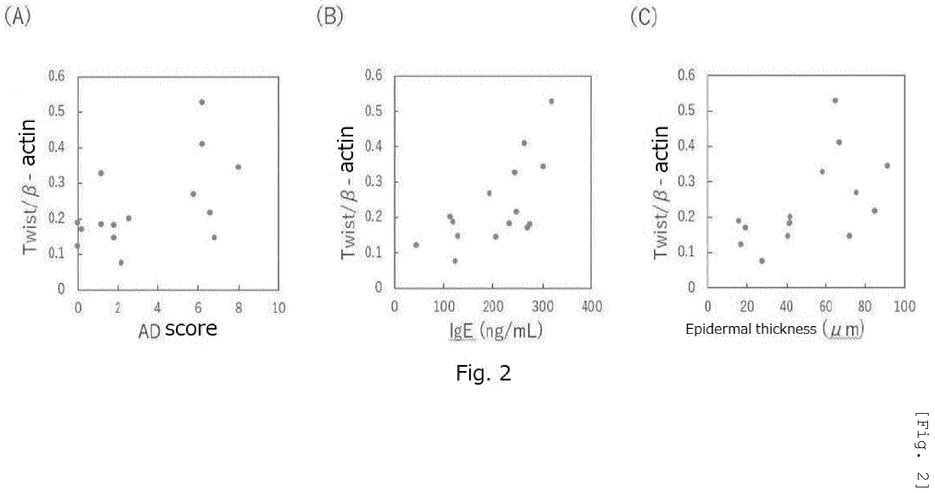

Fig. 2 shows the correlation between Twist/β-actin and the severity of atopic dermatitis ((A) AD score, (B) IgE, and (c) epidermal thickness), as measured in Example 1. The vertical axis indicates the Twist/β-actin value. The horizontal axis indicates the index value of the severity of atopic dermatitis.

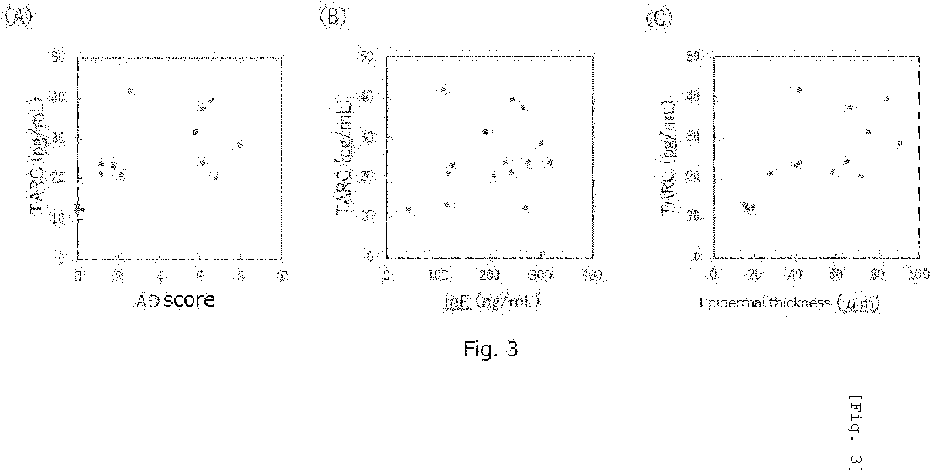

Fig. 3 shows the correlation between blood TARC concentration and the severity of atopic dermatitis ((A) AD score, (B) IgE, and (c) epidermal thickness), as measured in Comparative Example 1. The vertical axis indicates the blood TARC concentration. The horizontal axis indicates the index value of the severity of atopic dermatitis.

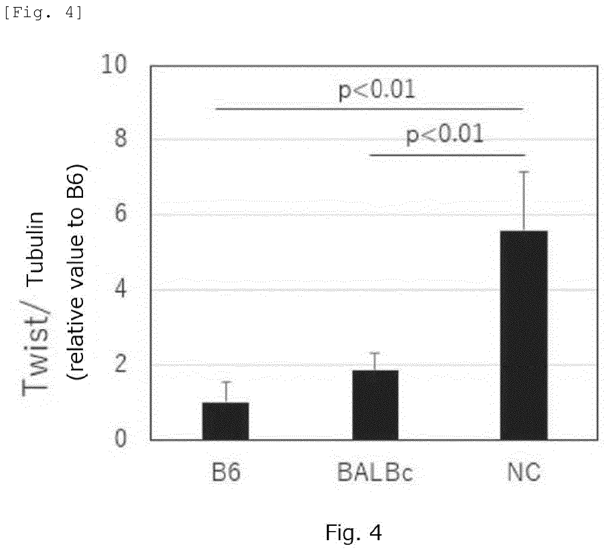

Fig. 4 shows the results of comparing Twist/tubulin values in normal animals and atopic dermatitis model animals, as measured in Example 2. The vertical axis indicates the relative value, with the Twist/tubulin value in C57BL/6 mice set at 1.

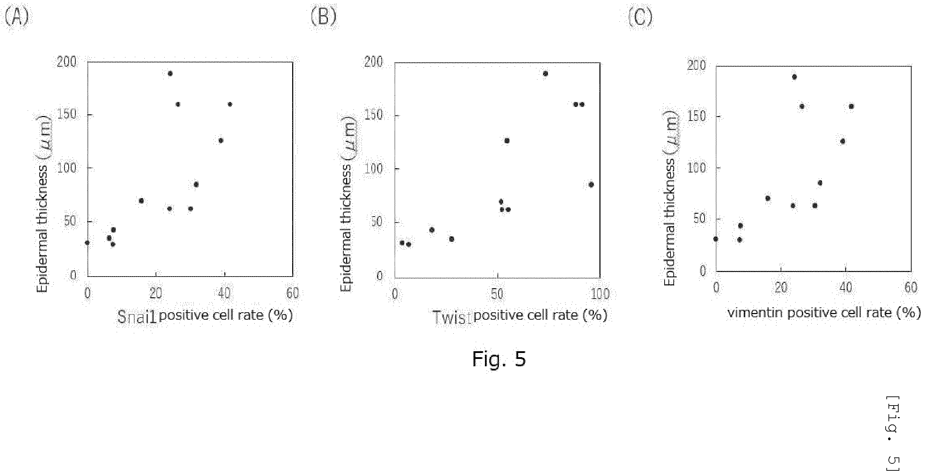

Fig. 5 shows the correlation between epithelial-mesenchymal transition marker (Snail, Twist, or vimentin) positive cells and epidermal thickness, as measured in Example 3. The vertical axis indicates epidermal thickness (um), and the horizontal axis indicates the rate of epithelial-mesenchymal transition marker-positive cells (%).

### MODE FOR CARRYING OUT THE INVENTION

### (Definitions)

It should be understood that terms used in this specification are used in the same manner as commonly used in the art unless otherwise specified. Therefore, unless defined otherwise, all technical and scientific terms used herein have the same meaning as commonly understood by those skilled in the art to which this invention belongs. In case of conflict, the present specification, including definitions, will control.

In the present specification, the expressions "contain" and "comprise" include the concepts of "contain", "include", "consist essentially of" and "consist only of".

In the present specification, the expression "and/or" includes the meaning of either the selection of "and" or the selection of "or". In other words, the expression "A and/or B" includes both the meanings of "A or B" and "A and B".

As used herein, "marker" refers to a substance that serves as an indicator of whether or not a certain condition (for example, the likelihood of the presence of an inflammatory skin disease, the severity, and/or degree of progression of the disease) is present. Such substances include genes, gene products, metabolic substances, enzymes, and the like.

As used herein, "epithelial-mesenchymal transition marker" refers to a marker expressed by cells in the process in which epithelial cells, such as epidermis cells, lose their cell polarity and cell adhesion function with surrounding cells and acquire migration and invasion capabilities, thereby changing into mesenchymal-like cells, such as fibroblasts.

As used herein, "inflammatory skin disease" includes skin diseases and disorders, and refers to a condition accompanied by a series of clinical signs and symptoms such as itching, edema, erythema, and peeling, and refers to a skin disease induced by various irritating factors that cause a series of inflammatory reactions in the skin. Clinical signs and symptoms of inflammatory skin diseases include ulceration, papules, inflammation, or blistering of the skin. Representative examples of inflammatory skin diseases include atopic dermatitis.

As used herein, "atopic dermatitis" refers to inflammatory dermatitis accompanied by prurigo, and is characterized by a chronic and recurrent course that repeats periods of remission and exacerbation. In many cases, the disease develops due to a family history of allergic asthma, allergic rhinitis (hay fever), allergic conjunctivitis, and the like, and/or a background of atopic predisposition, which is a predisposition to produce IgE antibodies.

As used herein, "subject" refers to the subject of testing in the method of the present disclosure, and examples of the subject include mammals (for example, humans, mice, rats, hamsters, rabbits, cats, dogs, cows, horses, sheep, monkeys, goats, pigs, and the like).

As used herein, "reference value" refers to a reference value for determining the presence or absence, likelihood of the presence, the severity, and/or degree of progression of an inflammatory skin disease by comparing the amount and/or concentration of a marker for epithelial-mesenchymal transition measured in a biological sample collected from a subject, and may be a measured value of a marker for epithelial-mesenchymal transition in a biological sample collected from a subject not having the target disease or a subject with a particular severity.

### 1.Method for testing presence or absence, likelihood of the presence, severity, and/or degree of progression of inflammatory skin disease

In one aspect, the present disclosure relates to a method for testing the presence or absence, likelihood of the presence, severity and/or degree of progression of an inflammatory skin disease, the method including a step of measuring the amount and/or concentration of an epithelial-mesenchymal transition marker in a biological sample collected from a subject. This method is described below.

The inflammatory skin disease is not particularly limited as long as it is a disease accompanied by inflammation of the skin and/or the onset process of which involves inflammation. Examples thereof include atopic dermatitis, psoriasis vulgaris, acne vulgaris, systemic lupus erythematosus, erythema nodosum, erythema multiforme, bullous disease, palmoplantar pustulosis, vasculitis, contact dermatitis, urticaria, drug eruption, prurigo, seborrheic dermatitis, nummular eczema, chronic lichen simplex, autosensitization dermatitis, stasis dermatitis, asteatotic eczema, granuloma annulare, keratosis pilaris, panniculitis, pyoderma gangrenosum, Stevens-Johnson syndrome, and toxic epidermis necrolysis, and preferably include atopic dermatitis.

The severity and degree of progression of the inflammatory skin disease are not particularly limited. The severity is based on grades such as mild, moderate, and severe, as well as evaluation scores such as the rash score given by a clinician (in the case of atopic dermatitis, Scoring Atopic Dermatitis (SCORAD) or EASI score). The degree of progression is a concept defined from the viewpoint of the pathology or progression of symptoms of the target disease, and can also be based on the above values. More specifically, the degree of progression may refer to the amount of change from a previously determined extent value and/or evaluation value. For example, if the disease was previously determined to be mild and then determined to be moderate one year later based on the method of the present disclosure, the progression from mild to moderate over the course of one year can be determined as the degree of progression. The degree of progression may be indicated as the amount of change in the evaluation value. Previously determined extent values and/or evaluation values may be determined based on conventional scores or markers, or may be determined based on the method of the present disclosure.

Scoring Atopic Dermatitis (SCORAD) refers to a score based on the extent of the rash (%), the severity of the rash (4-point scale from 0 to 3), and subjective symptoms (itching and lack of sleep each on an 11-point scale from 0 to 10).

The AD score used in Examples is a score used for animal test models, and refers to a score obtained by evaluating and assigning four items: (1) redness and bleeding on the back, (2) dry crust on the back, (3) ear edema, and (4) ear tissue loss on 4-point scale, namely, asymptomatic (0 points), mild (1 point), moderate (2 points), and severe (3 points) (Mina Yamamoto, et al., Allergology International 2007; 56: 139-148).

The subject is a target organism for the testing method of the present disclosure, and the organism species is not particularly limited. Examples of organism species of the subject include various mammals such as humans, monkeys, mice, rats, dogs, cats, and rabbits, and preferably humans.

The condition of target disease of the subject is not particularly limited. Examples of the subject include a subject in which the presence or absence of the target disease is unknown, a subject with no history of the target disease, a subject having a history of the target disease and having been treated for the target disease, a subject that has already been determined to have the target disease (or not) by other testing methods, and a subject in which the severity or degree of progression has been determined by target disease testing using interview methods, questionnaire testing methods, score methods, pathological diagnosis methods, blood biochemistry testing methods, and the like. When the subject is a human, regardless of the past medical history, any subject including a subject who seems to be a healthy subject can be the test subject. When a subject who is considered to be a healthy subject, it is effective for early detection and early diagnosis of a target disease in general medical examinations, complete medical examinations, and the like. In addition, when a subject in whom a suspicion of a target disease, the severity, and/or degree of progression have been diagnosed by a target disease test such as an interview method, a questionnaire test method, a score method, a pathological diagnosis method, and a blood biochemical test method, diagnosis of the target disease can be assisted by the test method of the present disclosure.

The epithelial-mesenchymal transition is a process in which epithelial cells such as epidermis cells lose the cell polarity and cell adhesion function thereof with surrounding cells, and acquire migration and infiltration ability, thereby changing into mesenchymal-like cells such as fibroblasts. The epithelial-mesenchymal transition plays an important role in various developmental processes including mesoderm formation, neural tube formation, and the like. In addition, the epithelial-mesenchymal transition is considered to appear in inflammation, wound healing, tissue fibrosis, cancer invasion, metastasis, and the like.

Examples of the epithelial-mesenchymal transition marker include E-cadherin, cytokeratin, ZO-1, mucin, surfactant protein, N-cadherin, vimentin, FSP-1, α-smooth muscle actin, type I collagen, ZEB1, ZEB2, Ovol1, Snail, and Slug, and preferably N-cadherin, vimentin, Twist, and Snail.

The biological sample is not particularly limited as long as it can contain a marker for epithelial-mesenchymal transition. Examples of the biological sample include samples derived from body fluids such as blood, urine, body secretion, nasal mucous membrane, oral mucous membrane, sweat, saliva, tears, and sputum, and samples derived from skin such as horny layer, epidermis, dermis, body hair, hair, hair follicle, sebaceous gland, and adipose tissue, and preferably include horny layer, epidermis, and hair follicle. The blood sample includes whole blood, plasma, serum, and the like, and can be prepared by appropriately treating whole blood collected from a subject. The horny layer can be directly collected from a mammal or the like in a minimally invasive manner using a tape stripping method, a collection method using a cotton swab, or the like. In addition, the collection is possible from the excised skin by a heat separation method. The epidermis can be collected by immersing the skin including the dermis in a NaBr solution or a hypertonic saline solution. The hair follicles can be collected directly from mammals or the like in a minimally invasive manner.

The method for measuring the amount and/or concentration of the epithelial-mesenchymal transition marker in the biological sample collected from the subject is not particularly limited, and examples thereof include an immunoassay method, a chromatography method, a mass spectrometry method, a nucleic acid aptamer method, and a quantitative PCR method. The immunoassay can be widely adopted regardless of a direct method, an indirect method, a homogeneous method, a heterogeneous method, a competitive method, a non-competitive method, and the like. More specific examples of the immunoassay include ELISA (for example, a direct method, an indirect method, a sandwich method, a competitive method, or the like), radioimmunoassay, immunoradiometric assay, enzyme immunoassay, sandwich EIA, immunochromatography, Western blot, immunoprecipitation, slot or dot blot assay, immune tissue staining, fluorescence immunoassay, and immunoassay using a surface plasmon resonance method. In addition, the amount and/or concentration of the epithelial-mesenchymal transition marker in the biological sample can be corrected on the basis of the wet and dry weights of the biological sample or the internal standard molecules (for example, β-actin, GAPDH, β2-microglobulin, tubulin, HPRT1) of the biological sample.

As one aspect, the test method of the present disclosure preferably includes a step of determining the presence or absence of the disease, the likelihood that the subject has the target disease, or the severity and/or degree of progression of the target disease in the subject based on the comparison result with the previously set reference value of the marker for epithelial-mesenchymal transition. Herein, "likelihood that the subject has the target disease" means "likelihood that the subject has the target disease at the time of biological sample collection", and "severity and/or degree of progression of the target disease" means "degree of the state of the target disease at the time of biological sample collection and change of the degree over time". "Likelihood that that the subject has the target disease at the time of biological sample collection" refers to the likelihood that the subject has the target disease determined based on the amount and/or concentration of the marker for epithelial-mesenchymal transition measured in the biological sample, and when the amount and/or concentration of the marker for epithelial-mesenchymal transition measured in the biological sample is higher than a reference value (for example, the amount and/or concentration of a marker of epithelial-mesenchymal transition measured in a healthy subject) means high likelihood that the subject has the target disease, and when the amount and/or concentration is equal to or less than the reference value means no or low likelihood that the subject has the target disease. "Degree of the state of the target disease at the time of biological sample collection" means the severity at the time of biological sample collection, the severity determined based on the amount and/or concentration of the marker for epithelial-mesenchymal transition measured in the biological sample, and "change in the degree over time" means a change from the degree of the state of the target disease determined in the past to the degree of the state of the target disease at the time of biological sample collection in the same subject.

The previously set reference value of the marker for epithelial-mesenchymal transition can be appropriately set by those skilled in the art from viewpoints such as sensitivity, specificity, positive predictive value, and negative predictive value. For example, when determining the likelihood that the subject has the target disease, the reference value can be set to the amount and/or concentration of the marker for epithelial-mesenchymal transition in a biological sample collected from a subject not having the target disease. More specifically, for example, the setting can be possible by performing statistical analysis (more specifically, a method using the Youden index is exemplified) based on analysis of a receiver operating characteristic (ROC) curve or the like using a value obtained by correcting the amount and/or concentration of the marker for epithelial-mesenchymal transition in biological samples collected from a subject having the target disease and a subject not having the target disease with an internal standard molecule or the like. It can be determined that when the amount and/or concentration of the marker for epithelial-mesenchymal transition in the biological sample collected from the subject is higher than the reference value, the subject has or may have the target disease, and when the amount and/or concentration is equal to or less than the reference value, the subject does not have the target disease or there is no or low likelihood that the subject has the target disease.

When determining the severity and/or degree of progression of the target disease, the reference value of the marker for epithelial-mesenchymal transition can be set using a biological sample collected from a subject with any severity and/or any degree of progression for the target disease. More specifically, for example, the setting is possible by performing statistical analysis based on analysis or the like of an ROC curve using a value obtained by correcting the amount and/or concentration of a marker for epithelial-mesenchymal transition in a subject having any severity and/or any degree of progression of the target disease with an internal standard molecule or the like. The severity indicator defined in the Atopic Dermatitis Clinical Practice Guidelines and other conventional indicators (for example, SCORAD, EASI score, conventional markers (for example, SCCA2, TARC, and IgE), or a combination thereof) may be used to determine the reference value for markers of epithelial-mesenchymal transition of the severity and/or degree of progression. For example, in order to determine the reference value for the severity and/or degree of progression of a target disease, a conventional indicator (for example, SCORAD, EASI score, conventional markers (for example, SCCA2, TARC, and IgE), or a combination thereof) can be correlated with the amount and/or concentration of a marker of epithelial-mesenchymal transition, and the reference value for each severity and/or degree of progression can be determined based on the correlation. More specifically, the severity and/or degree of progression can be determined by comparing the reference value with the amount and/or concentration of a marker for epithelial-mesenchymal transition measured in a biological sample collected from a subject, and determining to what extent the measured value corresponds to a conventional indicator (for example, corresponds to XX points on the EASI score), for example, based on the above-described correlation. Typically, the severity evaluation based on conventional atopic dermatitis scores and conventional markers (serological test values, median values) is as follows:
SCCA2: mild 2.0, moderate 3.5, and severe 10.8;
TARC: mild 885, moderate 897, and severe 2530; and,
IgG: mild 282, moderate 652, and severe 1869;
for the severity according to the guidelines for the treatment of atopic dermatitis, most severe: rash with severe inflammation is seen on 30% or more of the body surface; severe: rash with severe inflammation is seen on 10% or more but less than 30% of the body surface; moderate: rash with severe inflammation is seen on less than 10% of the body surface; and mild: only mild rash is observed regardless of the area;
SCORAD: evaluation with the following three items: the extent of rash (%), severity of rash (4-point scale from 0 to 3), and subjective symptoms (itching and lack of sleep each on an 11-point scale from 0 to 10) (maximum score is 103 points); and
EASI score: evaluation of severity in 4 body parts: head and neck, trunk, upper limbs, and lower limbs. The severity of the rash in each part (4-point scale from 0 to 3) and the area of the rash (7-point scale from 0 to 6) are evaluated, and the two are multiplied to calculate a score (maximum score is 72 points).

In another embodiment, a correlation diagram between the SCORAD or EASI score and the amount and/or concentration of the marker for epithelial-mesenchymal transition may be pre-prepared , and the SCORAD or EASI score points corresponding to the amount and/or concentration of the marker for epithelial-mesenchymal transition may be used as the severity and/or degree of progression.

The conventional severity evaluation for psoriasis vulgaris is as follows:
for relative body surface area (BSA), mild: less than 3%; moderate: 3 to 10%; and severe: over 10%;
PASI: skin symptoms (erythema, infiltration, desquamation) for each body part (head, upper limbs, trunk, lower limbs) are evaluated and scored on a 5-point scale (0: none, 1: mild, 2: moderate, 3: severe, and 4: extremely severe) (maximum score 72 points).

Conventional severity evaluation in acne vulgaris is as follows:
for skin findings, mild: 5 or less inflammatory skin rashes on one face; moderate: 6 or more and 20 or less inflammatory skin rashes on one face; severe: 21 or more and 50 or less inflammatory skin rashes on one face; and most severe: 51 or more inflammatory skin rashes on most one face.

Conventional severity evaluation in systemic lupus erythematosus is as follows:
for skin findings, mild: 5 or less inflammatory skin rashes on one face; moderate: 6 or more and 20 or less inflammatory skin rashes on one face; severe: 21 or more and 50 or less inflammatory skin rashes on one face; and most severe: 51 or more inflammatory skin rashes on one face,
for SLEDAI, score 1: fever, thrombocytopenia, and leukopenia; score 2: new skin eruption, alopecia, mucosal ulcer, pleurisy, nociculitis, low complement value, and increase in DNA binding capacity; score 4: arthritis, myositis, urine sedimentation, hematuria, proteinuria, and pyuria; score 8: spasm, psychiatric symptoms, organic brain syndrome, visual impairment, cranial nerve disorder, lupus headache, cerebrovascular disorder, and vasculitis (4 or more points are subjects for which medical expenses are granted) .

Further, it is preferable to include a step of determining the presence or absence of the target disease, likelihood that the subject has the disease, or the severity and/or degree of progression of the disease in the subject by comparing with the reference value of the marker for epithelial-mesenchymal transition.

### 2.Testing agent for target disease

In one aspect, the present disclosure relates to a testing agent for a target disease, including a detection reagent for an epithelial-mesenchymal transition marker (for example, at least one selected from the group consisting of an antibody or an antigen-binding fragment thereof against an epithelial-mesenchymal transition marker, and a functional nucleic acid). This will be described below. The functional nucleic acid may be a primer for amplifying at least a portion of the epithelial-mesenchymal transition marker.

The testing agent of the present disclosure is used to test the presence or absence of the target disease, likelihood of having the disease, the severity, and/or degree of progression of the disease.

When the testing agent of the present disclosure includes an antibody, a commercially available antibody or an antibody produced using, for example, hybridoma technology can be used. The testing agent of the present disclosure may be in the form of a composition including at least one selected from the group consisting of an antibody against an epithelial-mesenchymal transition marker. The composition may include other components as necessary. Examples of other components include bases, carriers, solvents, dispersants, emulsifiers, buffers, stabilizers, excipients, binders, disintegrants, lubricants, thickeners, humectants, colorants, fragrances, and chelating agents.

The testing agent of the present disclosure may be used in a kit including at least one selected from the group consisting of an antibody against an epithelial-mesenchymal transition marker. The kit may include an instrument, a reagent, and the like that can be used for performing the test method of the present disclosure. Examples of the instrument include a test tube, an ultrafiltration unit, and a chromatography column (for example, a Sephadex column). In addition, examples of the reagent include SDS and 2-mercaptoethanol.

When the testing agent of the present disclosure includes a gene quantification primer, a commercially available primer or, for example, a primer designed by the following method can be used.

### Primer design

Length: 17 to 25mer, GC content: 40 to 60%, Tm: 55 to 65°C, and sequence (A sequence with 3 or more consecutive G or C at the 3' end is avoided. A sequence in which the 3' end is T is avoided. A sequence that matches 2mer or more with the 3' end inside the primer is avoided.). Finally, BLAST searches are performed to confirm the specificity of the primer.

The testing agent of the present disclosure may be in the form of a composition including at least one selected from the group consisting of gene quantification primers for an epithelial-mesenchymal transition marker. In addition, the testing agent of the present disclosure may be used in a kit including at least one selected from the group consisting of gene quantification primers for an epithelial-mesenchymal transition marker.

### 3. Therapeutic agent for target disease

In the present disclosure, a method including the step of measuring the amount and/or concentration of an epithelial-mesenchymal transition marker in a biological sample collected from a subject can test likelihood of the presence of an inflammatory skin disease, in particular, atopic dermatitis, and the severity and/or degree of progression thereof. In addition, in the present disclosure, the severity of an inflammatory skin disease (for example, atopic dermatitis) and the amount and/or concentration of the epithelial-mesenchymal transition marker show a positive correlation, and thus the amount and/or concentration of the epithelial-mesenchymal transition marker can be used as an index of the severity of an inflammatory skin disease (for example, atopic dermatitis). In addition, when information on the severity of an inflammatory skin disease (for example, atopic dermatitis) is obtained according to the present invention, a pharmaceutical composition for suppressing epithelial-mesenchymal transition can be used as a therapeutic agent for an inflammatory skin disease, particularly atopic dermatitis. Examples of the molecule that suppresses epithelial-mesenchymal transition include EMT inhibitor-1. Without wishing to be bound by theory, epithelial cells play a role of a physical barrier in the skin and prevent invasion of substances and the like that cause inflammation, and in inflammatory skin diseases (for example, atopic dermatitis), when epithelial-mesenchymal transition occurs, epithelial cells lose a cell adhesion function, migrate and infiltrate to the surroundings, and change into mesenchymal-like cells such as fibroblasts, thereby losing the function of epithelial cells as a skin barrier and losing homeostasis of epithelial tissues. Therefore, in inflammatory skin diseases (for example, atopic dermatitis), a molecule that suppresses epithelial-mesenchymal transition is effective.

Other therapeutic agents include, but are not limited to, steroids, immunosuppressive agents, anti-allergic agents, anti-cytokine antibodies, anti-cytokine receptor antibodies, JAK inhibitors, and the like.

### EXAMPLES

Hereinafter, the invention of the present disclosure will be described in detail based on examples, but the invention of the present disclosure is not limited to these examples.

### Example 1. Correlation analysis between severity of atopic dermatitis and epithelial-mesenchymal transition marker

### (a) Preparation of atopic dermatitis model animal

Atopic dermatitis spontaneously occurring animal NC/Nga mice were obtained from The Jackson Laboratory Japan, Inc. The mouse is a model animal of atopic dermatitis, similar to human atopic dermatitis, most widely used in the field. NC/Nga mice (Male, 9 weeks old) were shaved under anesthesia with isoflurane (manufactured by FUJIFILM Wako Pure Chemical Corporation, catalog number: 099-06571), and 30 minutes after application of an epilat hair removal cream (manufactured by Kracie, Ltd.), 100 mg of a mite antigen Biostir AD (manufactured by Biostir Inc., catalog number: AD002) was applied to the back and auricle to sensitize the mite antigen. In the second and subsequent sensitization, 4% SDS was applied instead of the hair removal cream, and then 100 mg of Biostir AD was applied. A mite antigen was sensitized twice a week for three weeks (six times in total) to prepare an atopic dermatitis model animal.

### (b)Measurement of AD score

With respect to the prepared atopic dermatitis model animal, four items of (1) redness and bleeding of the back, (2) crustal dryness of the back, (3) edema of the auricle, and (4) tissue defect of the auricle were evaluated on 4-point scale, namely, asymptomatic (0 points), mild (1 point), moderate (2 points), and severe (3 points), and the total score thereof was taken as an AD score (Mina Yamamoto, et al., Allergology International 2007; 56: 139-148).

### (c)Measurement of blood IgE concentration

Blood was collected from an atopic dermatitis model animal, and the supernatant obtained by centrifugation at 3000 rpm for 15 minutes was used as plasma. The IgE concentration in the plasma was measured using IgE ELISA kit (manufactured by LBIS, catalog number: 639-02891).

### (d)Measurement of epidermal thickness

The skin of the collected atopic dermatitis model animal was immersed in Mildfor 10NM (manufactured by FUJIFILM Wako Pure Chemical Corporation, catalog number: 130-10527) at room temperature overnight and fixed. The fixed skin was dehydrated and paraffin-embedded, and then a skin section having a thickness of 5 µm was prepared using LEICA SM2010R (manufactured by Leica Biosystems Nussloch GmbH). The prepared skin section was deparaffinized, then hematoxylin and eosin stained, and then encapsulated using an encapsulant (manufactured by Merck KGaA, catalog number: 1.07961.0100). Then, images of hematoxylin-eosin stained sections were acquired using a BZ-X710 All-in-One Fluorescence Microscope (manufactured by KEYENCE CORPORATION), and the epidermal thickness was measured using image analysis software BZ-X analyzer (manufactured by KEYENCE CORPORATION).

### (e)Measurement of amount of epithelial-mesenchymal transition marker

The skin of the collected atopic dermatitis model animal was immersed in 2M sodium bromide at 37°C for 2 hours to peel off the epidermis. To the detached epidermis, 100 µL of RIPA buffer (1% NP-40, 20 mM Tris-HCl (pH 7.4), 0.15 M NaCl, 1 mM EDTA, phosphatase inhibitor cocktail (manufactured by Merck, catalog number: 11836170001), and protein inhibitor cocktail(manufactured by Merck KGaA, catalog number: 04906845001) was added, and the epidermis was chopped on ice, and then an epidermis suspension was prepared using a homogenizer pestle. Then, the epidermis suspension was centrifuged at 12000×g for 10 minutes at 4°C, and then the supernatant was recovered to provide an epidermis extract. The protein concentration of the epidermis extract was measured using a Protein assay kit (manufactured by NACALAI TESQUE, INC., catalog number: 06385-00) to prepare 500 µg/mL.

SDS sample buffer was added to and mixed with the epidermis extract, and the mixture was heated at 97°C for 3 minutes, and then subjected to 10% SDS-PAGE. Then, the gel was transferred to a PVDF membrane and blocked with Blocking one (manufactured by NACALAI TESQUE, INC., catalog number: 03953-95), and then a diluted anti-Snail antibody (Proteintech Group, Inc., catalog number 13099-1-AP), an anti-Twist antibody (manufactured by Abcam Limited, catalog number: ab50889), or an anti-β-actin antibody (manufactured by MEDICAL & BIOLOGICAL LABORATORIES CO., LTD., catalog number: M177-3) was added thereto and reacted overnight at 4°C. Then, the diluted HRP-labeled anti-mouse IgG antibody (manufactured by Cell Signaling Technology, Inc., catalog number: #7076) or HRP-labeled anti-rabbit IgG antibody (manufactured by Abcam Limited, catalog number: ab6721) was added to the washed PVDF membrane and reacted at room temperature for 1 hour. Then, a chemiluminescent reagent (manufactured by NACALAI TESQUE, INC., catalog number: 11644-40) was added to the washed PVDF membrane, and a signal was detected using ChemiDoc Touch Imaging System (manufactured by Bio-Rad Laboratories, Inc.). Each signal value was measured using image analysis software Image Lab (manufactured by Bio-Rad Laboratories, Inc.), and the amount of the epithelial-mesenchymal transition marker was calculated according to the following formula. Amount of epithelial-mesenchymal transition marker = signal value of epithelial-mesenchymal transition marker/β-actin signal value

### (e)Correlation analysis between severity of atopic dermatitis and amount of epithelial-mesenchymal transition marker

The correlation between the severity of atopic dermatitis (AD score, IgE, epidermal thickness) and the amount of the epithelial-mesenchymal transition markers in atopic dermatitis model animals was analyzed. As a result, as shown in Fig. 1 (Snail) and Fig. 2 (Twist), a correlation was shown between the severity of atopic dermatitis and the amount of the epithelial-mesenchymal transition markers. In addition, the correlation coefficient was calculated, and as shown in Table 1, a particularly high correlation was shown between the AD score and the amount of the epithelial-mesenchymal transition markers.

### [Table 1]

**Table 1**

| Index of severity | **AD** ^{score} | IgE | Epidermal thickness |
|---|---|---|---|
| Snail (correlation coefficient) | 0.81 | 0.53 | 0.84 |
| Twist (correlation coefficient) | 0.74 | 0.67 | 0.56 |
| TARC(correlation coefficient) | 0.60 | 0.22 | 0.64 |

Increased expression of Snail and Twist (both transcription factors) alters the expression of downstream genes controlled by Snail and Twist, inducing epithelial-mesenchymal transition (Hideyuki Saya, Journal of Familial Tumors, Vol. 10, No. 2, 2010). Therefore, it is likely that other markers associated with epithelial-mesenchymal transition other than Snail and Twist are also increased.

### Comparative Example 1. Measurement of existing markers in atopic dermatitis model animals

The TARC concentration in plasma of the atopic dermatitis model animals prepared in Example 1(a) was measured using a TARC ELISA kit (R&D Systems, Inc., catalog number: MCC170).

Analysis of the correlation between the severity of atopic dermatitis calculated in Example 1 (b to d) and the blood TARC concentration showed a certain degree of correlation between the severity of atopic dermatitis and the blood TARC concentration, as shown in Fig. 3. On the other hand, a comparison of correlation coefficients, as shown in Table 1, showed that the testing method of the present disclosure has a higher ability to test the severity of atopic dermatitis than the TARC testing method.

### Example 2. Comparison of epithelial-mesenchymal transition markers between normal animals and atopic dermatitis spontaneously occurring animals

Atopic dermatitis spontaneously occurring animal NC/Nga mice were obtained from The Jackson Laboratory Japan, Inc. In addition, C57BL/6 mice and BALB/c mice were obtained from CLEA Japan, Inc. as normal animals. For each mouse, the amount of Twist in the epidermis was measured according to the method described in Example 1(e), and the amount of internal standard in the epidermis was measured using an anti-tubulin antibody (Proteintech Group, Inc., catalog number: 11224-1-AP), and the amount of Twist in atopic dermatitis spontaneously occurring animals was compared with that in normal animals.

As a result, as shown in Fig. 4, atopic dermatitis spontaneously occurring animals showed significantly higher levels of the amount of epithelial-mesenchymal transition markers (p < 0.01) compared to normal animals, demonstrating that the testing method disclosed herein is highly capable of testing the likelihood of the presence of atopic dermatitis or the risk of developing atopic dermatitis in the future. When the amount of Twist was compared between normal animals (B6, Balbc) and atopic dermatitis model mice (NC), the amount of Twist was clearly increased in the latter. The amount of Twist increases in atopic dermatitis, and thus the amount of Twist is considered to increase during the process leading to atopic dermatitis. Therefore, setting the amount of Twist of healthy subjects as the minimum value and the amount of Twist of atopic dermatitis as the maximum value, it is possible to determine the risk of developing atopic dermatitis in the future based on the amount of Twist.

### Example 3. Correlation analysis between severity of human atopic dermatitis and epithelial-mesenchymal transition marker

### (a)Specimen of atopic dermatitis patient

Normal skin of a healthy subject (N = 3), skin of a non-lesion part and a lesion part of an atopic dermatitis patient (N = 4) were collected by biopsy.

### (b)Measurement of epidermal thickness

The collected skin was fixed, dehydrated, and paraffin embedded, and then a skin section was formed, and the epidermal thickness was measured in accordance with the method described in Example 1.(d).

### (c)Measurement of epithelial-mesenchymal transition marker rate

A skin section was deparaffinized and autoclaved at 120°C for 10 minutes, and then a 100 fold diluted mouse anti-Snai 1 antibody (manufactured by Santa Cruz Biotechnology, Inc., catalog number: SC-272977), a 50 fold diluted mouse anti-Twist antibody (manufactured by Abcam Limited, catalog number: ab50889), a 250 fold diluted rabbit anti-vimentin antibody (Cell Signaling Technology, Inc., catalog number: 5741), and a 250 fold diluted guinea pig anti-keratin 5 antibody (manufactured by OriGene Technologies, Inc., catalog number: BP5006) were added thereto and reacted overnight at 4°C. Each section was washed with PBS (-), then an Alexa 594 labeled anti-mouse antibody, a Dylight 564 labeled anti-rabbit antibody, and an Alexa 488 labeled anti-guinea pig antibody were added thereto and reacted at room temperature for 1 hour. Then, the washed section was subjected to nuclear staining, and then subjected to fluorescence observation using a BZ-X710 All-in-One Fluorescence Microscope, and an epithelial-mesenchymal transition marker rate (epithelial-mesenchymal transition marker positive cell rate) was calculated in accordance with the following formula. Epithelial-mesenchymal transition marker rate (epithelial-mesenchymal transition marker positive cell rate) (%) = number of epithelial-mesenchymal transition marker positive cells/number of keratin 5 positive cells

### (d)Correlation analysis between severity of atopic dermatitis and amount of epithelial-mesenchymal transition marker

The correlation between the severity of atopic dermatitis (epidermal thickness) and the amount of an epithelial-mesenchymal transition marker for a non-lesion part and a lesion part of healthy subjects and atopic dermatitis patients was analyzed. As a result, as shown in Fig. **5****,** a correlation was shown between the severity of atopic dermatitis and the amount of the epithelial-mesenchymal transition marker. In addition, as a result of calculating the correlation coefficient, as shown in Table **2,** a high correlation was shown between the severity of atopic dermatitis and the amount of the epithelial-mesenchymal transition marker in humans.

The epithelial-mesenchymal transition marker correlated with atopic dermatitis observed in mice in Examples 1 and 2 similarly correlated with atopic dermatitis in humans. Therefore, the results of the present example show that the epithelial-mesenchymal transition marker can be used as an index of atopic dermatitis in humans.

The marker rate (%) in the skin of healthy subjects was about 0 to about 5% for Snail, about 2 to about 20% for Twist, and about 0 to about 10% for Vimentin. On the other hand, the marker rate (%) in the skin of lesion part of atopic dermatitis was about 30 to about 40% for Snail, about 80 to about 90% for Twist, and about 30 to about 40% for vimentin.

### [Table 2]

**Table 2**

| Index of severity | Epidermal thickness |
|---|---|
| Snai1(correlation coefficient) | 0.71 |
| Twist(correlation coefficient) | 0.79 |
| Vimentin (correlation coefficient) | 0.71 |

This application claims the benefit of priority of Japanese Patent Application No. 2022-162538 filed on October 7, 2022 and Japanese Patent Application No. 2023-31988 filed on March 2, 2023, the contents of which are incorporated herein by reference.

## Claims

1. A method for analyzing a biological sample collected from a subject for testing presence or absence, likelihood of presence, severity, and/or degree of progression of an inflammatory skin disease in the subject, the method comprising a step of measuring an amount and/or concentration of an epithelial-mesenchymal transition marker in the biological sample collected from the subject.

2. The method according to claim **1,** wherein the inflammatory skin disease is atopic dermatitis.

3. The method according to claim 1 or **2,** wherein the epithelial-mesenchymal transition marker is at least one selected from the group consisting of N-cadherin, vimentin, Twist, and Snail.

4. The method according to any one of claims 1 to 3, further comprising a step of comparing a preset reference value with an amount and/or concentration of an epithelial-mesenchymal transition marker in a biological sample collected from the subject.

5. The method according to claim 4, wherein the reference value is an amount and/or concentration of an epithelial-mesenchymal transition marker in a biological sample collected from a subject not having an inflammatory skin disease.

6. The method according to any one of claims 1 to 5, wherein the biological sample is a sample of skin or derived from skin.

7. The method according to claim 6, wherein the sample derived from skin is at least one selected from the group consisting of a horny layer, an epidermis, and a hair follicle.

8. A testing agent comprising a detection reagent for an epithelial-mesenchymal transition marker, for testing presence or absence, likelihood of presence, severity, and/or degree of progression of an inflammatory skin disease in a subject.

9. The testing agent according to claim 8, wherein the detection reagent is selected from the group consisting of an antibody or an antigen-binding fragment thereof against the epithelial-mesenchymal transition marker, and a functional nucleic acid.

10. The testing agent according to claim 8 or 9, wherein the epithelial-mesenchymal transition marker is at least one selected from the group consisting of N-cadherin, vimentin, Twist, and Snail.

11. The testing agent according to any one of claims 8 to 10, wherein the inflammatory skin disease is atopic dermatitis.

12. A composition for determining presence or absence, likelihood of presence, severity, and/or degree of progression of an inflammatory skin disease in a subject, the composition comprising a reagent for measuring an amount and/or concentration of an epithelial-mesenchymal transition marker.

13. A composition for treating or preventing an inflammatory skin disease, the composition comprising a molecule that inhibits epithelial-mesenchymal transition.
